# FASCICULE DE BREVET EUROPEEN

(11) **EP 2 288 689 B1**
(45) Date de publication et mention de la délivrance du brevet: **16.05.2012**
(21) Numéro de dépôt: 09742421.2
(22) Date de dépôt: 30.04.2009
(51) Int. Cl.: C12N 1/04, B65B 25/00, B65B 31/02, B65B 39/04, B65D 81/20

(54) **PROCEDE DE CONDITIONNEMENT DE LEVURE SECHE**
VERPACKUNGSVERFAHREN FÜR TROCKENHEFE
METHOD FOR PACKAGING DRY YEAST

(30) Priorité: 06.05.2008 FR 0802501
(43) Date de publication de la demande: 02.03.2011
(73) Titulaire: Lesaffre et Compagnie, 75001 Paris (FR)
(72) Inventeur: MICHEL, Eric, F-59130 Lambersart (FR); MARCINIAK, Sophie, F-59700 Marcq en Baroeul (FR); HONORE, Alexis, F-59830 Bourghelles (FR); DEBRUYNE, Pascal, F-59700 Marcq en Baroeul (FR)
(74) Mandataire: Cabinet Plasseraud
(86) Numéro de dépôt international: PCT/IB2009/005436
(87) Numéro de publication internationale: WO 2009/136248

(56) Documents cités:
- EP-A- 1 184 298
- WO-A-2006/000065
- FR-A- 2 183 932
- FR-A- 2 900 639
- GB-A- 1 230 205
- US-A- 3 077 405
- US-A- 3 449 134

## Description

La présente invention se rapporte à un procédé de conditionnement de levure sèche dans un emballage fermé hermétiquement avec une teneur en oxygène résiduel réduite.

La levure de boulangerie peut être transportée et conservée sous forme liquide, sous forme comprimée ou sous forme asséchée. On entend par « levure sèche », de la levure contenant moins de 10% de matière humide et de préférence moins de 4 à 5% de matière humide. La levure sèche peut être préparée sous forme de granules ou vermicelles ou sous forme de poudre ou férule avant d'être conditionnée dans un emballage sous vide. Le conditionnement sous vide présente l'avantage de supprimer toute teneur en oxygène qui est cause d'une diminution rapide du pouvoir ferment de la levure sèche. Le conditionnement sous vide présente cependant l'inconvénient d'être onéreux. En outre, après une première ouverture, l'emballage sous vide est généralement difficile à refermer hermétiquement et la levure perd alors rapidement de son pouvoir ferment.

Le document FR-A-2 183 932 propose un procédé d'emballage de matières susceptibles d'une détérioration de leur propriétés utiles au cours du temps (notamment de la levure). Un gaz inerte est injecté dans une enveloppe contenant la matière active pour éjecter l'air de l'enveloppe. Après sorption partielle du gaz par la matière active, l'enveloppe est scellée pour faire apparaître une pression sub-atmosphérique dans l'emballage.

Le document GB-A-1 230 205 propose de conditionner de la levure sèche dans un emballage métallique ou plastique sous vide ou sous atmosphère d'azote.

Le document WO-A-2006/000065 concerne une composition en poudre contenant de la levure active conditionnée dans un emballage présentant au moins 5% d'atmosphère inerte après fermeture. La composition ne remplit pas complètement l'emballage qui présente un volume restant d'atmosphère inerte. La teneur en oxygène résiduel dans l'emballage est inférieure à 2% après fermeture. Ce document indique que l'emballage peut être scellé sous atmosphère d'azote mais ne précise pas comment cette atmosphère est crée ni maintenue tout au long du procédé de conditionnement.

Il existe donc un besoin pour un procédé de conditionnement de levure sèche qui puisse être mis en oeuvre à pression atmosphérique et qui permette de garantir une teneur en oxygène résiduel faible et de préférence inférieure à 4 % après fermeture de l'emballage pour une conservation optimale de la levure pendant au moins 2 ans.

A cet effet, l'invention propose de contrôler précisément l'atmosphère avant, pendant et après le remplissage du conteneur jusqu'à la fermeture hermétique de l'emballage.

Plus particulièrement, l'invention propose un procédé de conditionnement de levure sèche dans un emballage, le procédé comprenant les étapes consistant à:
- injecter du gaz inerte dans un conteneur destiné à recevoir la levure sèche ;
- injecter du gaz inerte dans une station de dosage de la levure sèche ;
- injecter du gaz inerte dans le conteneur contenant une dose de levure sèche avant fermeture étanche de l'emballage.

Selon les modes de réalisation, le procédé selon l'invention peut comprendre une ou plusieurs des caractéristiques suivantes :
- injecter du gaz inerte dans un réservoir de levure sèche ;
- le gaz inerte est injecté en plusieurs points de la station de dosage ;
- le gaz inerte est injecté en au moins un parmi trois points disposés en entrée et de part et d'autre d'un doseur de la station de dosage ;
- le gaz inerte est injecté en au moins un parmi trois points disposés en entrée et de part et d'autre d'une trémie de la station de dosage ;
- injecter du gaz inerte dans un système d'aspiration de poussière associé à la station de dosage ;
- diffuser du gaz inerte autour du conteneur entre le dosage de la levure et la fermeture de l'emballage ;
- maintenir une atmosphère inerte dans une enceinte regroupant un injecteur de gaz inerte dans le conteneur avant remplissage, la station de dosage et une rampe de diffusion de gaz inerte après remplissage du conteneur ;
- le gaz injecté est choisi parmi du dioxyde de carbone, de l'azote, de l'argon ou un mélange de ceux-ci ;
- injecter du gaz inerte avant fermeture étanche de l'emballage consiste à introduire de l'azote liquide ou de la neige carbonique dans le conteneur ;
- le débit du gaz injecté est supérieur à 0,7 m³/h ;
- là dose de levure sèche introduite dans le conteneur occupe 50 % à 95% du volume du conteneur après fermeture.

L'invention propose aussi un équipement pour le conditionnement de levure sèche dans un emballage, l'équipement comprenant :
- un injecteur adapté à injecter du gaz inerte dans un conteneur destiné à recevoir de la levure sèche ;
- une station de dosage de la levure sèche ;
- un injecteur adapté à injecter du gaz inerte dans la station de dosage ;
- une station de fermeture étanche de l'emballage ;
- un injecteur adapté à injecter du gaz inerte dans le conteneur avant fermeture de l'emballage.

Selon les modes de réalisation, l'équipement selon l'invention peut comprendre une ou plusieurs des caractéristiques suivantes :
- un injecteur adapté à injecter du gaz inerte dans un réservoir de levure sèche ;
- la station de dosage présente une pluralité de points d'injection de gaz inerte répartis sur une trémie et un doseur ;
- la station de dosage comprend au moins un parmi trois points d'injection de gaz inerte disposés en entrée et de part et d'autre du doseur ;
- la station de dosage comprend au moins un parmi trois points d'injection de gaz inerte disposés de part et d'autre de la trémie ;
- un injecteur adapté à injecter du gaz inerte dans un système d'aspiration de poussière associé à la station de dosage ;
- une rampe de diffusion adaptée à diffuser du gaz inerte autour du conteneur entre la station de dosage et la station de fermeture ;
- une enceinte à atmosphère inerte contrôlée regroupant l'injecteur de gaz inerte dans le conteneur avant remplissage, la station de dosage et la rampe de diffusion.

D'autres caractéristiques et avantages de l'invention apparaîtront à la lecture de la description qui suit, donnée à titre d'exemple et en référence aux figures qui montrent :
- Figure 1, une vue schématique d'un emballage ;
- Figure 2, un schéma d'une installation pour la mise en oeuvre du procédé de conditionnement selon l'invention ;
- Figure 3, un schéma du système de dosage de l'installation de la figure 2.

L'invention propose un procédé de conditionnement de levure sèche dans un emballage. L'emballage ainsi obtenu contient moins de 6 % d'oxygène résiduel après fermeture et préférentiellement moins de 4 %. On entend par « oxygène résiduel » la quantité d'oxygène dans la phase gazeuse contenue dans l'emballage fermé. Le pouvoir ferment de la levure n'est donc pas détérioré et la levure sèche ainsi conditionnée peut être conservée longtemps dans son emballage hermétiquement fermé.

De manière générale, les pertes de pouvoir ferment de la levure sont de deux types. D'une part les pertes dites oxydative qui sont proportionnelles à la quantité d'oxygène disponible dans l'emballage ; et d'autre part les pertes dites non oxydatives qui sont fonction de la durée et de la température de conservation. Plus la température de conservation est élevée, plus les pertes de pouvoir ferment sont importantes ; et plus le pourcentage d'oxygène résiduel présent dans l'emballage est important, plus les pertes de pouvoir ferment sont importantes.

Par exemple, des essais de simulation de conservation ont été réalisés avec un emballage dont la perméabilité aux gaz est de 0,003 cm³.m².24h à une température de l'ordre de 26°C. Ces essais ont montré une conservation acceptable de la levure pendant 12 mois si le pourcentage d'oxygène initial dans l'emballage est inférieur ou égal à 6 % ; et une conservation acceptable pendant 24 mois si le pourcentage d'oxygène initial dans l'emballage est inférieur ou égal à 4 %. Dans ces exemples d'essais, on a considéré comme acceptable une perte de pouvoir ferment de la levure de l'ordre de 20% après 12 mois (respectivement 24 mois).

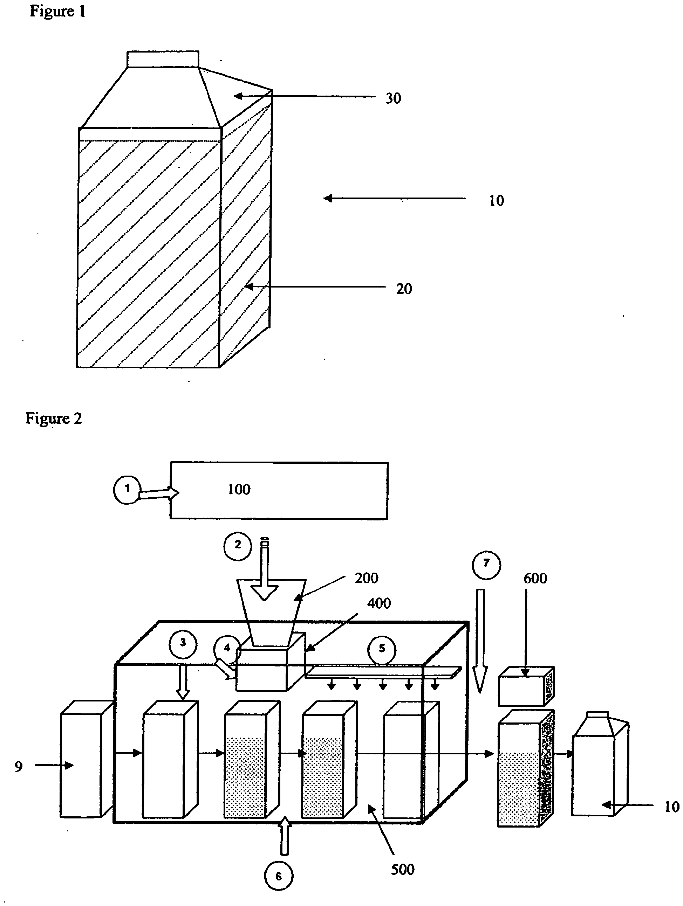

La figure 1 illustre schématiquement un tel emballage 10. L'emballage peut présenter une forme générale de boîte avec un fond, des parois latérales et un dessus hermétiquement fermé, par soudure par exemple. La boîte formant l'emballage peut être constituée d'une feuille multicouche conformée en conteneur par pliage et collage ou soudure. La feuille multicouche peut comprendre notamment de l'aluminium, du polyester, du polypropylène, du polyéthylène et/ou du polyamide. L'emballage peut être une boîte de type Tetrapak® par exemple. Un tel emballage est facile à ouvrir ; une amorce de déchirure de la soudure peut par exemple être prévue. Un tel emballage peut aussi être muni de moyen de fermeture après une première ouverture, par exemple un bouchon vissé ou clippé fermé d'un opercule étanche avant la première ouverture.

L'emballage 10 est destiné à conditionner de la levure sèche à pression atmosphérique. La levure 20, éventuellement mélangée avec des additifs de saveurs, d'émulsifiants ou autres, occupe la majeur partie de l'emballage ; au moins 50 % du volume de l'emballage. Le haut de l'emballage présente cependant une zone vide de produit. De fait, comme l'emballage 10 n'est pas fermé sous vide, un espace résiduel 30 est présent dans l'emballage après fermeture ; cet espace résiduel constitue la phase gazeuse contenue dans l'emballage fermé. L'espace résiduel 30 occupe environ 5 % ou plus du volume de l'emballage fermé. Dans le cas d'un emballage 10 contenant de la levure sèche 20, il est important que l'espace résiduel 30 soit composé d'une atmosphère artificielle sèche avec une teneur faible en oxygène. On cherche notamment à avoir une teneur en oxygène résiduel inférieur à 4 % du volume gazeux de l'emballage fermé.

A cet effet, l'invention propose un procédé de conditionnement de levure sèche dans lequel l'atmosphère est précisément contrôlée avant, pendant et après le remplissage du conteneur jusqu'à la fermeture hermétique de l'emballage.

Le schéma de la figure 2 illustre une installation pour la mise en oeuvre d'un tel procédé de conditionnement.

La figure 2 montre un réservoir 100 de levure sèche. Une quantité donnée de levure sèche est prélevée de ce réservoir 100 par une station de dosage 200 pour être introduite dans un conteneur 9 qui est ensuite hermétiquement scellé sur une station de fermeture 600 pour former un emballage fermé 10.

L'invention propose de contrôler l'atmosphère de conditionnement en injectant du gaz inerte avant, pendant et après le remplissage du conteneur.

Un injecteur de gaz 3 est ainsi prévu pour injecter du gaz inerte dans le conteneur 9 avant remplissage afin de ne pas mélanger de la levure avec une atmosphère riche en oxygène. Un injecteur de gaz 2 est également prévu pour injecter du gaz inerte dans la station de dosage 200 afin de limiter l'introduction d'oxygène au moment de l'introduction de la levure dans le conteneur.

Afin de limiter encore l'introduction d'oxygène avec la levure lors du remplissage du conteneur 9, du gaz inerte peut être injecté 1 dans le réservoir de levure 100 au fur et à mesure du soutirage de la levure depuis le réservoir 100. En outre, un autre injecteur de gaz 4 peut aussi être prévu au niveau d'un système d'aspiration des poussières 400 annexé à la station de dosage 200 ; la levure entraîne le gaz inerte ainsi injecté dans sa chute depuis la station de dosage 200 vers le conteneur 9.

Le procédé de l'invention propose aussi d'injecter du gaz inerte juste avant la fermeture étanche de l'emballage. Un injecteur de gaz 7 est ainsi prévu avant la station de fermeture 600. La fermeture étanche de l'emballage 10 peut se faire par soudure de couches polyester de la feuille multicouche formant le conteneur. Une telle soudure peut être réalisée par insufflation d'air chaud par exemple. L'air chaud est cependant fortement chargé en oxygène. Afin de pallier les effets de la soudure par air chaud, de l'azote liquide 7 peut être versé dans le conteneur contenant une dose de levure. L'expansion de l'azote liquide va permettre de chasser efficacement l'air encore présent dans le haut du conteneur avant fermeture étanche du conteneur pour former un emballage 10 hermétique. D'autres modes de fermeture étanche peuvent être envisagés sans sortir du cadre de l'invention, comme par exemple une soudure par thermo-résistance ou une soudure aux ultrasons. De la neige carbonique peut également être utilisée en lieu et place de l'azote liquide ou en complément.

La figure 2 montre aussi une enceinte 500 regroupant l'injecteur de gaz 3 du conteneur 9 vide, la station de dosage 200 avec le système d'aspiration des poussières 400 et une rampe de diffusion de gaz inerte 5. Cette rampe de diffusion de gaz 5 permet de diffuser du gaz inerte au dessus et autour du conteneur déjà rempli par une dose de levure mais pas encore hermétiquement fermé. Cette rampe de diffusion permet ainsi le maintien d'une atmosphère pauvre en oxygène dans le conteneur avant fermeture. Une injection de gaz inerte 6 est également prévue dans l'enceinte 500 afin de pallier l'introduction d'oxygène apporté par les mouvements des conteneurs. L'atmosphère dans l'enceinte 500 peut être contrôlée pour maintenir un taux d'oxygène de l'ordre de 15 %.

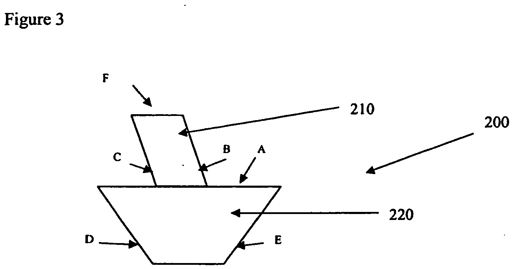

La figure 3 montre un schéma du système de dosage 200. On cherche à réduire au maximum le taux d'oxygène contenu dans la levure elle-même avant son introduction dans le conteneur 9 car l'oxygène apporté par la levure dans le conteneur est difficile à éliminer sur la ligne de conditionnement. Afin de réduire au maximum le taux d'oxygène apporté par la levure elle-même lors du dosage, il est proposé d'injecter du gaz inerte en plusieurs points de la station de dosage. Sur la figure 3, six points d'injection sont illustrés mais il est entendu que seul un ou certains de ces points ou encore d'autres peuvent être utilisés lors de la mise en oeuvre du procédé de l'invention.

La station de dosage 200 comprend une trémie 210 et un doseur 220. Peuvent ainsi être prévus un point d'injection de gaz inerte A en entrée du doseur 220 et deux points d'injection D, E de part et d'autre du doseur 220, ainsi que deux points d'injection de gaz inerte B, C de part et d'autre de la trémie 210 et un point d'injection de gaz F en entrée de la trémie.

Le gaz injecté peut être du dioxyde de carbone CO₂, de l'azote N₂ ou tout autre gaz inerte tel que de l'argon Ar.

Le dioxyde de carbone CO₂ est un gaz plus lourd que l'azote ; il peut être avantageusement utilisé pour l'injection de gaz inerte dans le conteneur avant et après dosage (points d'injection 3, 4 et 5 notamment) car il restera dans le conteneur plus longtemps que l'azote du fait de sa densité. L'azote N₂ sera de préférence utilisé pour le réservoir 100, la station de dosage 200 et l'enceinte 500 (points d'injection 1, 2 et 6 notamment) pour des raisons de sécurité. Il est cependant entendu qu'un mélange de ces gaz peut également être utilisé à chacun ou certains des points d'injection. Pour l'injection de gaz 7 au niveau de la station de fermeture étanche 600, on choisira de préférence de l'azote liquide ou une pastille de neige carbonique afin de bénéficier de l'effet d'expansion du gaz pour chasser efficacement l'oxygène du haut du conteneur au moment de la fermeture.

Le débit du gaz injecté doit être suffisant pour assurer un remplacement significatif de l'air ambiant par le gaz inerte injecté. Dans le cas d'une unique alimentation en gaz, le débit peut être régulé entre les différents points d'injection. On cherchera à garantir un débit supérieur à 0,7 m³/h en tout point d'injection avec un débit nettement supérieur (supérieur à 2 m³/h) aux points d'injection dans le conteneur avant remplissage 3 et au niveau de la station de dosage 2, 4.

On peut également contrôler la vitesse de déplacement du conteneur entre la station de remplissage 200 et la station de fermeture 600 afin de s'assurer que la dose de levure sèche introduite dans le conteneur reste en contact avec le gaz inerte pendant quelques fractions de secondes avant fermeture étanche du conteneur.

Le procédé de l'invention permet le conditionnement de levure sèche dans un emballage à pression atmosphérique contenant moins de 4 % d'oxygène dans le volume gazeux de l'emballage après fermeture. Un tel procédé permet une conservation optimale de la levure sur une longue durée.

Un exemple de conditionnement de levure sèche dans un emballage a été mené dans les conditions suivantes.

Le taux d'oxygène résiduel est mesuré à l'aide d'un appareil OXI-BOX Mobile 02-gasanalyser de la société Rycobelgroup et selon la procédure fournie par le fabricant. L'emballage utilisé est un multicouches comprenant de l'extérieur vers l'intérieur une couche de polyéthylène, une couche en carton, une couche en aluminium de 6,35 µm d'épaisseur et une couche de polyéthylène. Cet emballage est fourni par la société Elopak et offre une perméabilité à l'oxygène inférieure à 0,05 cm³.m².24h. Le bouchon de l'emballage peut être un bouchon classique soudé par l'extérieur ou un bouchon type « Pure Twist » d'Elopak soudé par l'extérieur. Un bouchon de type « Pure Twist » est préféré car il peut être soudé sans altérer la couche d'aluminium de l'emballage.

Une première série d'essais de conditionnement a été mise en oeuvre avec 6 points de gazage.

Avant d'être conditionnée en emballage, la levure doit présenter un taux d'oxygène le plus bas possible. Si la levure n'est pas « gazée » avant introduction dans le conteneur 9, elle apportera un volume d'oxygène difficile à éliminer sur la ligne de production. On a donc une première injection d'azote au point de gazage 1 au niveau du réservoir 100 et une deuxième injection d'azote 2 en 5 points au niveau de la station de dosage 200.

On a également un point de gazage 3 avant remplissage du conteneur 9, afin de ne pas mélanger la levure à une atmosphère riche en oxygène. On a encore un point de gazage 4 au niveau de la boîte d'aspiration des poussières 400 pendant le remplissage. La levure entraîne le CO₂ dans sa chute dans le conteneur 9.

On prévoit aussi un point de gazage 5 après le remplissage du conteneur 9, par une rampe de diffusion de manière à maintenir une atmosphère pauvre en oxygène dans le conteneur non encore hermétiquement fermé.

Une injection d'azote 6 est aussi prévue en deux points dans l'enceinte 500 regroupant les points d'injection 3, 4 et 5 afin que le volume d'air déplacé lors du transport du conteneur 9 présente un taux d'oxygène le plus bas possible.

Ces premiers essais ont conduit de manière répétitive à un taux d'oxygène résiduel inférieur à 4 %. Ces résultats sont obtenus soit avec un système de soudure par insufflation d'air chaud (chargé en oxygène) soit avec un système de soudure par thermo-compression.

Une deuxième série d'essais de conditionnement a été mise en oeuvre avec 7 points de gazage. Les 6 points de gazage décrits en référence à la première série ont été maintenus, auxquels s'est ajoutée une injection d'azote 7 au niveau de la station de fermeture 600, afin de pallier aux effets du système de soudure par apport d'air chaud et pour s'assurer un taux d'oxygène le plus bas possible dans le haut de l'emballage avant sa fermeture. Les résultats montrent qu'une dose d'azote liquide de 76 ms permet de baisser le taux d'oxygène résiduel dans l'emballage à 2,2 % et qu'une dose d'azote liquide de 176 ms permet d'atteindre un taux d'oxygène résiduel dans l'emballage de 1,8 %.

Les modes de réalisation décrits ci-dessus et les figures doivent être considérés comme ayant été présentés à titre illustratif et non restrictif, et l'invention n'est pas censée être limitée aux détails fournis ici mais peut être modifiée en restant dans le cadre de la portée des revendications annexées. En particulier, d'autres points d'injections de gaz inerte peuvent être prévus que ceux illustrés.

## Revendications

1. Un procédé de conditionnement de levure sèche dans un emballage (10), le procédé comprenant les étapes consistant à:
- injecter du gaz inerte (3) dans un conteneur (9) destiné à recevoir la levure sèche ;
- injecter du gaz inerte (2) dans une station de dosage (200) de la levure sèche ;
- injecter du gaz inerte (7) dans le conteneur contenant une dose de levure sèche avant fermeture étanche (600) de l'emballage.

2. Le procédé de la revendication 1, comprenant en outre une étape consistant à injecter du gaz inerte (1) dans un réservoir (100) de levure sèche.

3. Le procédé de la revendication 1 ou 2, dans lequel le gaz inerte est injecté en plusieurs points de la station de dosage (200).

4. Le procédé de la revendication 3, dans lequel le gaz inerte est injecté en au moins un parmi trois points (A, D, E) disposés en entrée et de part et d'autre d'un doseur (220) de la station de dosage (200).

5. Le procédé de la revendication 3 ou 4, dans lequel le gaz inerte est injecté en au moins un parmi trois points (B, C, F) disposés en entrée et de part et d'autre d'une trémie (210) de la station de dosage (200).

6. Le procédé de l'une des revendications 1 à 5, comprenant en outre une étape consistant à injecter du gaz inerte (4) dans un système d'aspiration de poussière (400) associé à la station de dosage (200).

7. Le procédé de l'une des revendications 1 à 6, comprenant en outre une étape consistant à diffuser du gaz inerte (5) autour du conteneur entre le dosage (200) de la levure et la fermeture (600) de l'emballage.

8. Le procédé de l'une des revendications 1 à 7, comprenant en outre une étape consistant à maintenir une atmosphère inerte (6) dans une enceinte (500) regroupant un injecteur de gaz inerte dans le conteneur avant remplissage, la station de dosage (200) et une rampe de diffusion de gaz inerte après remplissage du conteneur.

9. Equipement pour le conditionnement de levure sèche dans un emballage (10), l'équipement comprenant :
- un premier injecteur (3) adapté à injecter du gaz inerte dans un conteneur (9) destiné à recevoir de la levure sèche ;
- une station de dosage (200) de la levure sèche ;
- un deuxième injecteur (2) adapté à injecter du gaz inerte dans la station de dosage ;
- une station de fermeture étanche (600) de l'emballage ;
- un troisième injecteur (7) adapté à injecter du gaz inerte dans le conteneur avant fermeture de l'emballage.

10. L'équipement de la revendication 9, comprenant en outre un injecteur (1) adapté à injecter du gaz inerte dans un réservoir (100) de levure sèche.

11. L'équipement de la revendication 9 ou 10, dans lequel la station de dosage (200) présente une pluralité de points d'injection de gaz inerte répartis sur une trémie (210) et un doseur (220).

12. L'équipement de la revendication 11, dans lequel la station de dosage (200) comprend au moins un parmi trois points d'injection de gaz inerte (A, D, E) disposés en entrée et de part et d'autre du doseur (220).

13. L'équipement de la revendication 11 ou 12, dans lequel la station de dosage (200) comprend au moins un parmi trois points d'injection de gaz inerte (B, C, F) disposés de part et d'autre de la trémie (210).

14. L'équipement de l'une des revendications 11 à 13, comprenant en outre un injecteur (4) adapté à injecter du gaz inerte dans un système d'aspiration de poussière (400) associé à la station de dosage (200).

15. L'équipement de l'une des revendications 9 à 14, comprenant en outre une rampe de diffusion (5) adaptée à diffuser du gaz inerte autour du conteneur entre la station de dosage (200) et la station de fermeture (600).

16. L'équipement de la revendication 15, comprenant en outre une enceinte (500) à atmosphère inerte contrôlée regroupant l'injecteur (3) de gaz inerte dans le conteneur avant remplissage, la station de dosage (200) et la rampe de diffusion (5).

## Claims

1. A method for packaging a dry yeast in a package (10), the method comprising the following steps:
- injecting inert gas (3) into a container (9) intended to receive the dry yeast;
- injecting inert gas (2) into a dosing station (200) of the dry yeast;
- injecting inert gas (7) into the container containing a dose of dry yeast before sealed closing (600) of the package.

2. The method according to claim 1, also comprising a step consisting of injecting inert gas (1) into a dry yeast reservoir (100).

3. The method according to claim 1 or 2, wherein the inert gas is injected at several points of the dosing station (200).

4. The method according to claim 3, wherein the inert gas is injected into at least one of three points (A, D, E) positioned at the inlet and on either side of a dosimeter (220) of the dosing station (200).

5. The method according to claim 3 or 4, wherein the inert gas is injected into at least one of three points (B, C, F) positioned at the inlet and on either side of a hopper (210) of the dosing station (200).

6. The method according to one of claims 1 to 5, also comprising a step consisting of injecting inert gas (4) into a dust aspiration system (400) associated with the dosing station (200).

7. The method according to one of claims 1 to 6, also comprising a step consisting of diffusing inert gas (5) around the container between the dosing (200) of the yeast and the closing (600) of the package.

8. The method according to one of claims 1 to 7, also comprising a step consisting of maintaining an inert atmosphere (6) in an enclosure (500) grouping together an inert gas injector for injecting gas into the container before filling, the dosing station (200) and a diffusion ramp for diffusing inert gas after filling of the container.

9. Equipment for packaging dry yeast in a package (10),
the equipment comprising:
- a first injector (3) adapted to inject inert gas into a container (9) intended to receive dry yeast;
- a dosing station (200) for the dry yeast;
- a second injector (2) adapted to inject inert gas into the dosing station;
- a station for sealably closing (600) the package;
- a third injector (7) adapted to inject inert gas into the container before closing the package.

10. The equipment according to claim 9, also comprising an injector (1) adapted to inject inert gas into a dry yeast reservoir (100).

11. The equipment according to claim 9 or 10, wherein the dosing station (200) has a plurality of points for injecting inert gas distributed on a hopper (210) and a dosimeter (220).

12. The equipment according to claim 11, wherein the dosing station (200) comprises at least one of three inert gas injection points (A, D, E) positioned at the inlet and on either side of the dosimeter (220).

13. The equipment according to claim 11 or 12, wherein the dosing station (200) comprises at least one of three inert gas injection points (B, C, F) positioned on either side of the hopper (210).

14. The equipment according to one of claims 11 to 13, also comprising an injector (4) adapted to inject inert gas into a dust aspiration system (400) associated with the dosing station (200).

15. The equipment according to one of claims 9 to 14, also comprising a diffusion ramp (5) adapted to diffuse inert gas around the container between the dosing station (200) and the closing station (600).

16. The equipment according to claim 15, also comprising an enclosure (500) with a controlled inert atmosphere grouping together the injector (3) for injecting inert gas into the container before filling, the dosing station (200) and the diffusion ramp (5).

## Patentansprüche

1. Verfahren zum Verpacken von Presshefe in einer Verpackung (10), wobei das Verfahren die folgenden Schritte umfasst, darin bestehend:
- inertes Gas (3) in einen Behälter (9) einzuleiten, der dazu bestimmt ist, die Presshefe aufzunehmen,
- inertes Gas (2) in eine Dosieranlage (200) für die Presshefe einzuleiten,
- inertes Gas (7) in den Behälter, der eine Presshefedosis enthält, vor dem dichten Verschluss (600) der Verpackung einzuleiten.

2. Verfahren nach Anspruch 1, ferner umfassend einen Schritt, der darin besteht, inertes Gas (1) in einen Presshefebehälter (100) einzuleiten.

3. Verfahren nach Anspruch 1 oder 2, bei dem das inerte Gas an mehreren Punkten der Dosieranlage (200) eingeleitet wird.

4. Verfahren nach Anspruch 3, bei dem das inerte Gas an mindestens einem von drei Punkten (A, D, E) eingeleitet wird, die am Eingang und beiderseits eines Dosiergeräts (220) der Dosieranlage (200) angeordnet sind.

5. Verfahren nach Anspruch 3 oder 4, bei dem das inerte Gas an mindestens einem von drei Punkten (B, C, F) eingeleitet wird, die am Eingang und beiderseits eines Trichters (210) der Dosieranlage (200) angeordnet sind.

6. Verfahren nach einem der Ansprüche 1 bis 5, ferner umfassend einen Schritt, der darin besteht, inertes Gas (4) in ein mit der Dosieranlage (200) verbundenes Staubsaugsystem (400) einzuleiten.

7. Verfahren nach einem der Ansprüche 1 bis 6, ferner umfassend einen Schritt, der darin besteht, inertes Gas (5) um den Behälter zwischen der Dosierung (200) der Hefe und dem Verschluss (600) der Verpackung zu verteilen.

8. Verfahren nach einem der Ansprüche 1 bis 7, ferner umfassend einen Schritt, der darin besteht, eine inerte Atmosphäre (6) in einem Raum (500) aufrecht zu erhalten, der eine Einleitvorrichtung für inertes Gas in den Behälter vor der Befüllung, die Dosieranlage (200) und eine Rampe zur Verteilung von inertem Gas nach der Befüllung des Behälters umfasst.

9. Ausrüstung zur Verpackung von Presshefe in einer Verpackung (10), wobei die Ausrüstung folgendes umfasst:
- eine erste Einleitvorrichtung (3), die geeignet ist, inertes Gas in einen Behälter (9) einzuleiten, der dazu bestimmt ist, Presshefe aufzunehmen;
- eine Dosieranlage (200) für die Presshefe;
- eine zweite Einleitvorrichtung (2), die geeignet ist, inertes Gas in die Dosieranlage einzuleiten;
- eine Vorrichtung zum dichten Verschluss (600) der Verpackung;
- eine dritte Einleitvorrichtung (7), die geeignet ist, inertes Gas in den Behälter vor dem Verschluss der Verpackung einzuleiten.

10. Ausrüstung nach Anspruch 9, ferner umfassend eine Einleitvorrichtung (1), die geeignet ist, inertes Gas in einen Presshefebehälter (100) einzuleiten.

11. Ausrüstung nach Anspruch 9 oder 10, bei der die Dosieranlage (200) eine Vielzahl von Punkten zum Einleiten von inertem Gas umfasst, die auf einem Trichter (210) und einem Dosiergerät (220) verteilt sind.

12. Ausrüstung nach Anspruch 11, bei der die Dosieranlage (200) mindestens eine von drei Punkten zum Einleiten von inertem Gas (A, D, E) umfasst, die am Eingang und beiderseits des Dosiergeräts (220) angeordnet sind.

13. Ausrüstung nach Anspruch 11 oder 12, bei der die Dosieranlage (200) mindestens einen von drei Punkten zum Einleiten von inertem Gas (B, C, F) umfasst, die beiderseits des Trichters (210) angeordnet sind.

14. Ausrüstung nach einem der Ansprüche 11 bis 13, ferner umfassend eine Einleitvorrichtung (4), die geeignet ist, inertes Gas in ein Staubsaugsystem (400), das mit der Dosieranlage (200) verbunden ist, einzuleiten.

15. Ausrüstung nach einem der Ansprüche 9 bis 14, ferner umfassend eine Verteilungsrampe (5), die geeignet ist, inertes Gas um den Behälter zwischen der Dosieranlage (200) und der Verschlussvorrichtung (600) zu verteilen.

16. Ausrüstung nach Anspruch 15, ferner umfassend einen Raum (500) mit kontrollierter inerter Atmosphäre, umfassend die Einleitvorrichtung (3) von inertem Gas in den Behälter vor der Befüllung, die Dosieranlage (200) und die Verteilungsrampe (5).
